# EUROPEAN PATENT SPECIFICATION

(11) **EP 1 488 237 B1**
(45) Date of publication and mention of the grant of the patent: **11.05.2011**
(21) Application number: 03713156.2
(22) Date of filing: 26.03.2003
(51) Int. Cl.: G01N 33/543, G01N 21/55

(54) **METHOD, SYSTEM AND COMPUTER PROGRAM FOR DETECTING MOLECULAR BINDING INTERACTIONS COMPRISING RESPONSE CURVE QUALITY CONTROL**
VERFAHREN, SYSTEM UND COMPUTERPROGRAMM ZUM NACHWEIS MOLEKULARER BINDUNGSWECHSELWIRKUNGEN MIT REAKTIONSKURVEN-QUALITÄTSKONTROLLE
PROCEDE, SYSTEME ET PROGRAMME DE DETECTION DES INTERACTIONS DE LIAISON MOLECULAIRE IMPLIQUANT LE CONTROLE DE QUALITE EXPRIME PAR DES COURBES DE REPONSE

(30) Priority: 27.03.2002 SE 0200949; 27.03.2002 US 367806 P
(43) Date of publication of application: 22.12.2004
(73) Proprietor: GE Healthcare Bio-Sciences AB, 751 84 Uppsala (SE)
(72) Inventor: ANDERSSON, Karl, S-756 43 Uppsala (SE); BORG, Peter, S-754 47 Uppsala (SE)
(74) Representative: Stavbom, Ellen Elisabet
(86) International application number: PCT/SE2003/000500
(87) International publication number: WO 2003/081245

(56) References cited:
- WO-A-97/06418
- WO-A1-97/09618
- DE-A1- 10 005 301
- US-A- 5 242 828
- US-A- 5 955 729
- DATABASE WPI Week 200049, Derwent Publications Ltd., London, GB; Class B04, AN 2000-535299, XP002981199 & JP 2000 180453 A (TOSHIBA KK) 30 June 2000

## Description

### TECHNICAL FIELD

The present invention relates to a method of analysing molecular binding interactions at a sensing surface, and more particularly to the quality control of the resulting data describing the molecular interactions. The invention also relates to an analytical system including such a quality control as well as to a computer program for performing the method and a computer readable medium containing the program.

### BACKGROUND OF THE INVENTION

Analytical sensor systems that can monitor interactions between molecules, such as biomolecules, in real time are gaining increasing interest. These systems are often based on optical biosensors and usually referred to as interaction analysis sensors or biospecific interaction analysis sensors. A representative such biosensor system is the Biacore^{®} instrumentation sold by Biacore AB (Uppsala, Sweden) which uses surface plasmon resonance (SPR) for detecting interactions between molecules in a sample and molecular structures immobilized on a sensing surface. With the Biacore^{®} systems it is possible to determine in real time without the use of labeling, and often without purification of the substances involved, not only the presence and concentration of a particular molecule in a sample, but also additional interaction parameters such as, for instance, the association rate and dissociation rate constants for the molecular interaction. The Biacore^{®} system is currently used in life science research as well as in the drug discovery industry and in food analysis.

A typical output from the Biacore^{®} and similar biosensor systems is a graph or curve describing the progress of the molecular interaction with time. This curve, which is usually displayed on a computer screen, is often referred to as a "sensorgram".

While so far it has readily been possible for the operator of the biosensor instrument to assess the quality of the produced sensorgrams manually and discard any sensorgram of unacceptable quality, the current trend towards systems with ever increasing throughput and information density in the analyses performed puts a more and more heavy burden on the operator. There is therefore a need for means that facilitate the quality assessment in biosensor systems, especially where large sets of sensorgrams are produced.

In WO9706418, a method for measuring the concentration of an analyte present in a biological fluid is disclosed. The method includes the steps of applying NIR radiation to calibration samples to produce calibration data, analyzing calibration data to identify and remove outliers, constructing a calibration model, collecting and analyzing unknown samples to identify and remove outliers, and predicting analyte concentration of non-outliers from the calibration model. Analysis of calibration data includes data pretreatment, data decomposition to remove redundant data, and identification and removal of outliers using generalized distances.

### SUMMARY OF THE INVENTION

It is an object of the present invention to improve the operation of a biosensor system of the type referred to above by providing means for data processing of especially a large set of detection curves to classify the curves with regard to quality. Such quality classification may particularly be used to identify curves which differ from that of the majority of the curves studied and therefore potentially are of bad quality.

Therefore, in one aspect, the present invention provides a method of analysis, wherein molecular, particularly biomolecular, interactions at one or more sensing surface areas are detected and respective response curves representing the progress of each interaction with time are produced. According to the invention, a resulting set of response curves is subjected to a quality assessment procedure comprising the steps of:
a) selecting at least one quality-related parameter for the response curves, and for each different parameter defining at least one quality descriptor,
b) computing for each response curve in the set thereof, values for the different quality descriptors,
c) based on the values for the different quality descriptors, computing for each response curve a quality classification indicative of the quality of the response curve in relation to all response curves of the set,
d) selecting response curves having deviating quality classifications, and
e) subjecting the selected response curves to a validation procedure to determine whether a response curve or curves are to be rejected or not.

In another aspect, the present invention provides an analytical system for studying molecular interactions, which comprises data processing means for classifying the response curves with regard to quality.

In still another aspect, the present invention provides a computer program product comprising program code means for performing the method.

In yet another aspect, the present invention provides a computer program product comprising program code means stored on a computer readable medium for performing the method.

### BRIEF DESCRIPTION OF THE DRAWINGS

Figure 1 is a sensorgram showing the interaction between a sample and a target molecule.
Figure 2 shows two acceptable (left) and two unacceptable (right) sensorgrams.
Figure 3 is a flow chart showing the steps in an exemplary embodiment of the present invention.
Figure 4 is an overlay plot of five good and four bad sensorgrams with disturbances indicated at A, B, C and D.
Figure 5 is an illustration of a sensorgram where long term changes have been climinated by a filter, whereas short term fluctuations at B and C are retained.

### DETAILED DESCRIPTION OF THE INVENTION

As mentioned above, the present invention relates to analytical sensor methods, particularly biosensor based, where molecular interactions are studied and the results are presented in real time, as the interactions progress, in the form of detection curves, often called sensorgrams.

Biosensors may be based on a variety of detection methods. Typically such methods include, but are not limited to, mass detection methods, such as piezoelectric, optical, thermo-optical and surface acoustic wave (SAW) device methods, and electrochemical methods, such as potentiometric, conductometric, amperometric and capacitance methods. With regard to optical detection methods, representative methods include those that detect mass surface concentration, such as reflection-optical methods, including both internal and external reflection methods, angle, wavelength or phase resolved, for example ellipsometry and evanescent wave spectroscopy (EWS), the latter including surface plasmon resonance (SPR) spectroscopy, Brewster angle refractometry, critical angle refractometry, frustrated total reflection (FTR), evanescent wave ellipsometry, scattered total internal reflection (STIR), optical wave guide sensors, evanescent wave-based imaging such as critical angle resolved imaging, Brewster angle resolved imaging, SPR angle resolved imaging, and the like. Further, photometric methods based on, for example, evanescent fluorescence (TIRF) and phosphorescence may also be employed, as well as waveguide interferometers.

The presently most commonly used detection principle is surface plasmon resonance (SPR) spectroscopy. An exemplary type of SPR-based biosensors is sold by Biacore AB (Uppsala, Sweden) under the trade name BIACORE^{®} (hereinafter referred to as "the BIACORE instrument"). These biosensors utilize a SPR based mass-sensing technique to provide a "real-time" binding interaction analysis between a surface bound ligand and an analyte of interest.

The BIACORE instrument includes a light emitting diode (LED), a sensor chip including a glass plate covered with a thin gold film, an integrated fluid cartridge providing a liquid flow over the sensor chip, and a photo detector array. Incoming light from the LED is totally internally reflected at the glass/gold interface and detected by the photo detector array. At a certain angle of incidence ("the SPR angle"), a surface plasmon wave is set up in the gold layer which is detected as an intensity loss "or dip" in the reflected light. More particularly, and as is appreciated by those skilled in the art, the phenomenon of SPR associated with the BIACORE instrument is dependent on the resonant coupling of monochromatic p-polarized light, incident on a thin metal film via a prism and a glass plate, to oscillations of the conducting electrons, called plasmons, at the metal film on the other side of the glass plate. These oscillations give rise to an evanescent field which extends a distance of the order of one wavelength (≈1 µm) from the surface into the liquid flow. When resonance occurs, light energy is lost to the metal film through a collective excitation of electrons therein and the reflected light intensity drops at a sharply defined angle of incidence, the SPR angle, which is dependent on the refractive index within reach of the evanescent field in the proximity of the metal surface.

As noted above, the SPR angle depends on the refractive index of the medium close to the gold layer. In the BIACORE instrument, dextran is typically coupled to the gold surface, with the analyte-binding ligand being bound to the surface of the dextran layer. The analyte of interest is injected in solution form onto the sensor surface through the fluid cartridge. Because the refractive index in the proximity of the gold film depends on (i) the refractive index of the solution (which is constant), and (ii) the amount of material bound to the surface, the binding interaction between the bound ligand and analyte can be monitored as a function of the change in SPR angle.

A detailed discussion of the technical aspects of the BIACORE instrument and the phenomenon of SPR may be found in U.S. Patent No. 5,313,264. More detailed information on matrix coatings for biosensor sensing surfaces is given in, for example, U.S. Patents Nos. 5,242,828 and 5,436,161. In addition, a detailed discussion of the technical aspects of the biosensor chips used in connection with the BIACORE instrument may be found in U.S. Patent No. 5,492,840. The full disclosures of the above-mentioned U.S. patents are incorporated by reference herein.

A typical output from the BIACORE instrument is a "sensorgram", which is a plot of response (measured in "resonance units" or "RU") as a function of time. An increase of 1,000 RU corresponds to an increase of mass on the sensor surface of about 1 ng/mm². As sample containing an analyte contacts the sensor surface, the ligand bound to the sensor surface interacts with the analyte in a step referred to as "association." This step is indicated on the sensorgram by an increase in RU as the sample is initially brought into contact with the sensor surface. Conversely, "dissociation" normally occurs when sample flow is replaced by, for example, a buffer flow. This step is indicted on the sensorgram by a drop in RU over time as analyte dissociates from the surface-bound ligand.

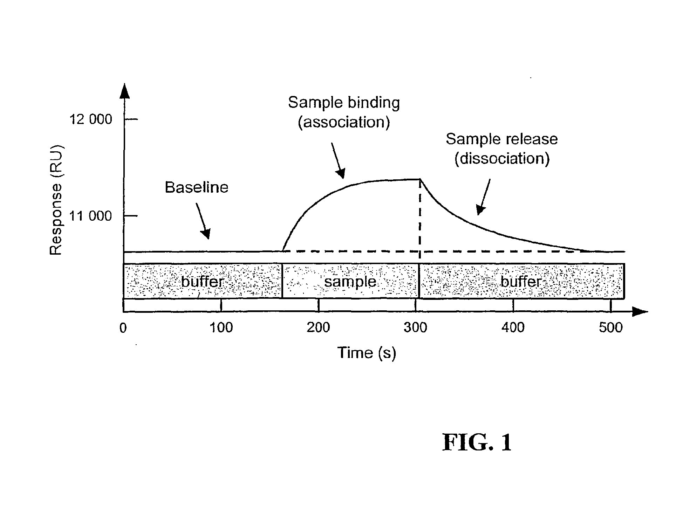

A representative sensorgram for the BIACORE instrument is presented in Figure 1, which depicts a sensing surface having an immobilized ligand (e.g. an antibody) interacting with analyte in a sample. The y-axis indicates the response (here in resonance units (RU)) and the x-axis indicates the time (here in seconds). Initially, buffer is passed over the sensing surface giving the "baseline response" in the sensorgram. During sample injection, an increase in signal is observed due to binding of the analyte (i.e., association) to a steady state condition where the resonance signal plateaus. At the end of sample injection, the sample is replaced with a continuous flow of buffer and a decrease in signal reflects the dissociation, or release, of analyte from the surface. The slope of the association/dissociation curves provides valuable information regarding the interaction kinetics, and the height of the resonance signal represents surface concentration (i.e., the response resulting from an interaction is related to the change in mass concentration on the surface).

The detection curves, or sensorgrams, produced by biosensor systems based on other detection principles will have a similar appearance.

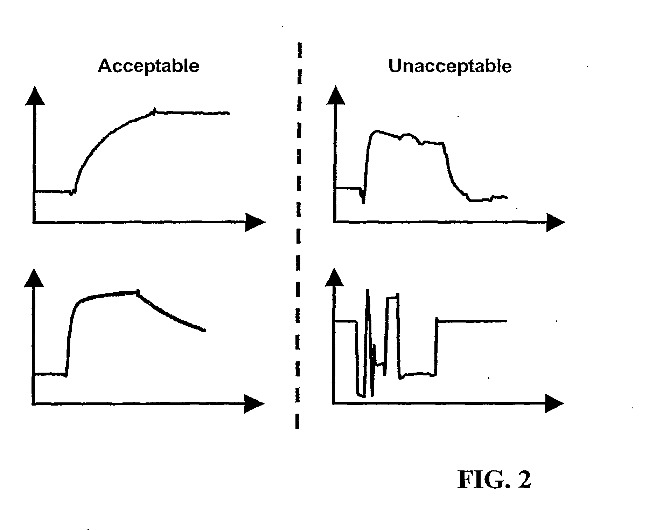

Sometimes the sensorgrams produced may for various reasons be of unacceptable quality and therefore have to be discarded. Figure 2 shows examples of two acceptable and two unacceptable sensorgrams. The two curves to the left are both acceptable. The top-right curve, on the other hand, is too unstable, and the bottom-right curve is deformed due to air-peaks (air bubbles in the fluid flow). Today, a control of the quality of sensorgrams is normally done by the user making an overlay plot of the curves to be analyzed and visually searching for oddities in the curves.

The current trend in biosensor systems is, however, a development towards high throughput systems capable of producing large sets of sensorgrams in a relatively short time. It is readily seen that already with a moderate increase in throughput, it will be impracticable for the user to inspect all the sensorgrams one at a time for assessing the quality thereof.

According to the present invention this problem is overcome by providing for data processing of the sensorgrams to at least substantially assist the user in assessing their quality. An algorithm has been devised, which is applicable in situations where a large set of sensorgrams is studied and classified with regard to quality, for example to identify curves with an odd quality, i.e. which differs from the quality of most of the sensorgrams in the set. The quality of a sensorgram being odd does, however, not necessarily mean that the quality is bad, and the "odd" sensorgrams are therefore subjected to a validation procedure where it is decided if the sensorgram is to be accepted or discarded. The validation procedure includes the use of at least one decision support. One such decision support is ocular (visual) inspection of the sensorgrams. Another decision support includes information on the reason why a sensorgram has been classified as odd. Still another decision support includes information on "time clusters" of odd sensorgrams, i.e. many sensorgrams associated with a specific time period or periods when the sensorgrams were produced. Using one or more of these decision supports, the operator (user) manually removes unaccepted sensorgrams. The validation procedure may also comprise an automated decision support in the form of a "decision algorithm" replacing any manual operation. The procedure of data processing of remaining sensorgrams and inspection of identified odd sensorgrams by the user is then repeated in an iterative manner until no more unacceptable sensorgrams are identified.

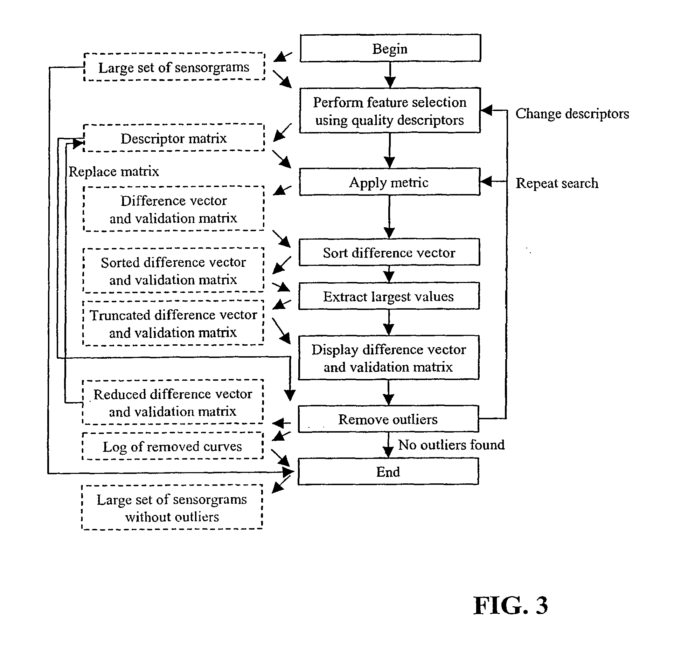

A flow chart of an embodiment of the algorithm is shown in Figure 3. This algorithm is designed to remove curves with a quality different from most of a large set of sensorgrams, so-called "outliers", and basically comprises the steps of (i) representing the sensorgrams with a number of quality descriptors, (ii) applying a quality classification method to the descriptors to find outliers, and (iii) removing the outliers. Preferably, a semi-supervised iterative approach is used.

The process is started with a large set of sensorgrams, usually more than about 100, for example in the range of from about 1000 to about 4000, obtained by running a number of test cycles on a biosensor system, such as, e.g., the BIACORE instrument.

The first step is to select the sensorgram features (curve parameters) used to determine the quality of the sensorgrams. Examples of such features are baseline slope, air spikes, and carry-over between measurements, just to mention a few. While it may be possible to use only a small number of features, such as e.g. three to five different features, it is usually preferred to use at least ten or fifteen different features. Each selected feature of a sensorgram is given a value, herein referred to as a "quality descriptor", which, for example, may be a numerical value or a vector.

The descriptors of each sensorgram are then transformed to a vector of descriptor values. In this way each sensorgram has been reduced to a set of descriptor values representing the different quality parameters. Thus, instead of the sensorgram, there are now a small number of figures in a vector which describe only the properties of interest of the sensorgram. The descriptor vectors for all the sensorgrams in the set are collected in a descriptor matrix.

A quality metric, usually an equation, is then applied to the descriptor matrix to estimate the difference in quality between each sensorgram and the rest of the sensorgrams in the set. This translates the descriptor matrix to a difference vector (containing differences) and validation matrix (containing estimates of the contribution to the difference of each descriptor).

The difference vector is then sorted with regard to difference magnitude to obtain a sorted difference vector and validation matrix.

A predetermined number of the largest difference values are extracted, e.g. the 50 or 100 largest values, to obtain a truncated difference vector and validation matrix, which is displayed to the user. It is understood that sensorgrams with large differences may be outliers with respect to the quality descriptors.

Usually, the user inspects the corresponding sensorgrams (or only a fraction thereof as desired), to decide which sensorgrams have insufficient quality, and removes them (manually) as outliers. As mentioned above and to be described in more detail below, the user may also utilize other types of decision supports. The removed outliers are collected in a log of removed curves, and the remaining sensorgrams (i.e. all sensorgrams minus removed outliers) are represented in a new descriptor matrix (replacing the original descriptor matrix). The search for outliers is repeated by again applying the quality metric equation and proceeding as described above to display the, e.g., 50 new sensorgrams that represent the largest differences. The reason for applying the quality metric equation again is that the metric may use the entire set of sensorgrams as a reference, and the set has changed. The process is repeated until the user cannot find any unacceptable, or bad, sensorgrams among those presented to him, the end result being a large set of sensorgrams without outliers.

As indicated in the flow chart, it is possible to change descriptors when repeating the search.

A basic characteristic of the present invention is the selection of curve quality features and their descriptors. Generally applicable quality features, or parameters, are odd curve shapes, such as baseline slope, spikes (e.g. an air spike during sample injection), oscillations and jumps. Other exemplary quality parameters include carry-over between measurements, binding to a reference surface area, and dissociation to a negative value (below zero). Suitable quality parameters for each particular situation may readily be selected by the skilled person.

Each quality parameter corresponds to one or more descriptors, a descriptor being a formula or algorithm that with one or more sensorgrams as input produces, for example, a numerical value as output. If, for instance, one of the descriptors is oscillations of the baseline, a sensorgram for which the baseline descriptor has the value 10 has a more oscillating baseline than a sensorgram where the descriptor has the value 5. A descriptor measuring the carry-over between measurements in the sensorgram is in its simplest form only a relative response (the response at the end of a buffer injection relative to the baseline level). An example of a descriptor table (matrix) is given in Table 1 below.

**Table 1**

| Cycle | Baseline slope | Carry-over | Air spike |
|---|---|---|---|
| 1 | 2.0 | 4.5 | 0.185 |
| 2 | 0.1 | 4.8 | 0.036 |
| 3 | 2.0 | 4.5 | 0.272 |
| 4 | 1.1 | 4.8 | 0.082 |
| 5 | 2.3 | 4.4 | 0.036 |

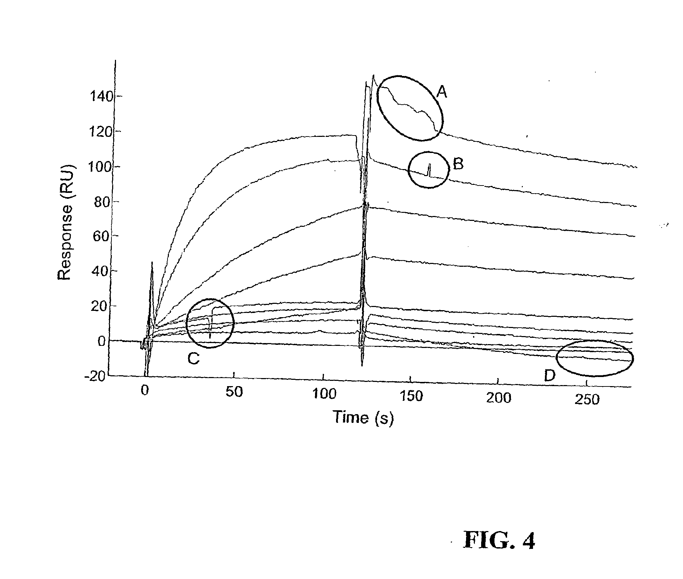

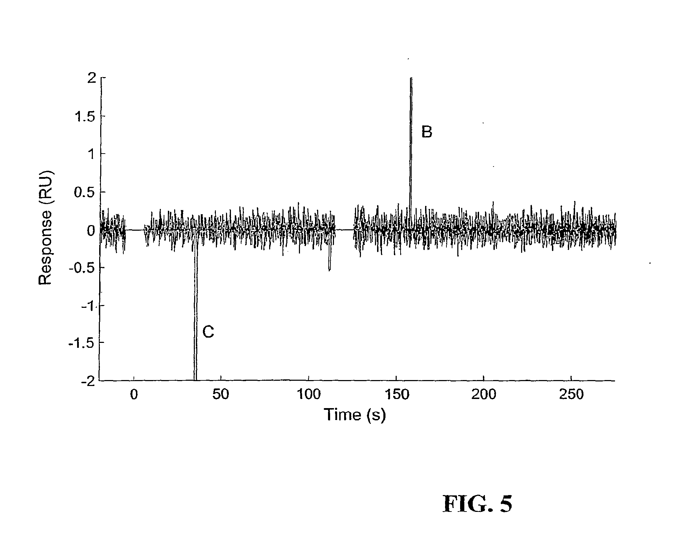

Another type of descriptor will be described with reference to Figures 4 and 5. While Figure 2 referred to above shows some examples of unacceptable sensorgrams, additional examples are given in Figure 4 which shows an overlay plot of five acceptable (good) and four unacceptable (bad) sensorgrams. As to the latter, A and B are affected by disturbances during dissociation, C has a discontinuity in the association phase, and D has a dissociation level less than zero.

Figure 5 illustrates the sensorgrams in Figure 4 after applying to each sensorgram a filter that eliminates longer term fluctuations while retaining short-term fluctuations. As seen in Figure 5, the maximum deviation from zero for the resulting curves are clearly largest for B and C. Utilizing this value as a primitive descriptor, B and C can be detected as different from the rest of the set.

Another basic characteristic of the present invention is the classification of the sensorgrams with regard to their quality by applying a quality classification method. Each sensorgram is represented by a descriptor vector, and the descriptor vectors are collected in a descriptor matrix. To classify the sensorgrams by quality, it is determined how similar the sensorgrams are to each other. The quality classification method may, for example, comprise the use of a quality metric, usually an equation, as described with regard to Fig. 3 above. Alternative classification methods include the use of a cluster algorithm, e.g. a KNN cluster algorithm, which classifies the sensorgrams in groups having a similar quality; a neural network or an expert system. All these quality classification methods are *per se* well-known to a person skilled in the art.

When, for example, a quality metric equation is used, each vector may be seen as a point in space, and the similarity between sensorgrams may then be represented by the distances between the respective points.

To measure the distances between the descriptor vectors, a statistical method may be used which measures the distance from each respective vector to all the other vectors seen as a group. Thereby each vector is reduced to a single value that describes how similar the descriptor vector is to all the other vectors. Sensorgrams having approximately the same value are then about equal qualitywise regarding the descriptors and the statistical method. Statistical methods that may be used include methods that are *per se* well known to the skilled person. Some specific exemplary methods are briefly described below.

"Mahalanobis distance" is a generalisation of the Euclidian distance between two points. Areas with a constant distance are ellipsoids centered around the mean value. When the descriptors are uncorrelated and the variances are equal to one in all directions, the areas are spheres and the Mahalanobis distance is equivalent to the Euclidian distance. The measure as such comprises a nonnalization of the descriptors by means of the inverse of the covariance matrix.

"Manhattan distance" sums up the descriptor vector.

"Principal component 1 vs 2" returns the score vectors 1 and 2 for the descriptor matrix. In contrast to the other methods mentioned above, this method does not provide any ranking.

The quality classification may include rescaling, or "normalizing", the descriptor values to make them comparable. An exemplary normalization method is the "mean centre" method, which sets the mean value of the descriptor values to zero. Other examples of normalization procedures are "mean centre and unit variance" (sets the mean value of the descriptors to zero and variance to one), and "unit variance" (sets variances to one).

As mentioned above, it is possible to change descriptors between reiterations of the outlier detection procedure (see Fig. 3). It is to be noted, however, that also the normalization and/or the quality classification method may be changed before each reiteration.

As also mentioned above, the user makes use of at least one "decision support" when validating the sensorgrams classified as odd. Thus, usually, the user obtains a visual plot of the classification result, and based thereon displays sensorgrams to be validated for possible removal. The user may, however, alternatively, or additionally, obtain information on which specific descriptor or descriptors that caused a particular classification of a sensorgram. He may also alternatively, or additionally, obtain information on time periods during the production of the sensorgrams to which many odd sensorgrams in a set may be related ("time clusters"). Alternatively, however, the whole validation procedure may be carried out by a decision algorithm without assistance by the user.

The above described quality assessment procedure is readily reduced to practice in the form of a computer system running software which implements the steps of the procedure. The invention also extends to computer programs, particularly computer programs on or in a carrier, adapted for putting the quality assessment procedure of the invention into practice. The carrier may be any entity or device capable of carrying the program.

While any suitable computer language may be used to implement the present invention, it is currently preferred to use a suite of MATLAB™ module files (The MathWorks, Inc., Natick, MA, U.S.A.).

The invention will be further illustrated by the following non-limiting Example.

### EXAMPLE

Eleven sensorgrams (below referred to as RU 1 to 11) were extracted from test data obtained with a BIACORE^{®} 3000 (Biacore AB, Uppsala, Sweden). These sensorgrams were used to successfully run a simple embodiment of the algorithm outlined in Fig. 3 in MATLAB 5.3.1.29215a (R11.1) (The MathWorks, Inc., Natick, MA, U.S.A.), using a PC with Windows NT 4.0. As descriptors were used (i) negative dissociation ("negd2"), (ii) jumpy association region ("assjmpmedian"), and (iii) jumpy dissociation region ("dissjmpmedian"). The metric used was the norm of the difference between the descriptors and the mean of the descriptor matrix. The program and the sensorgrams (values) are shown below.

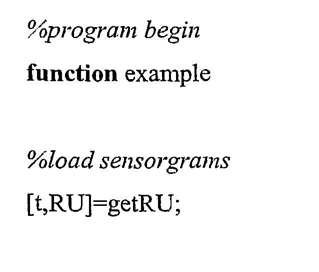

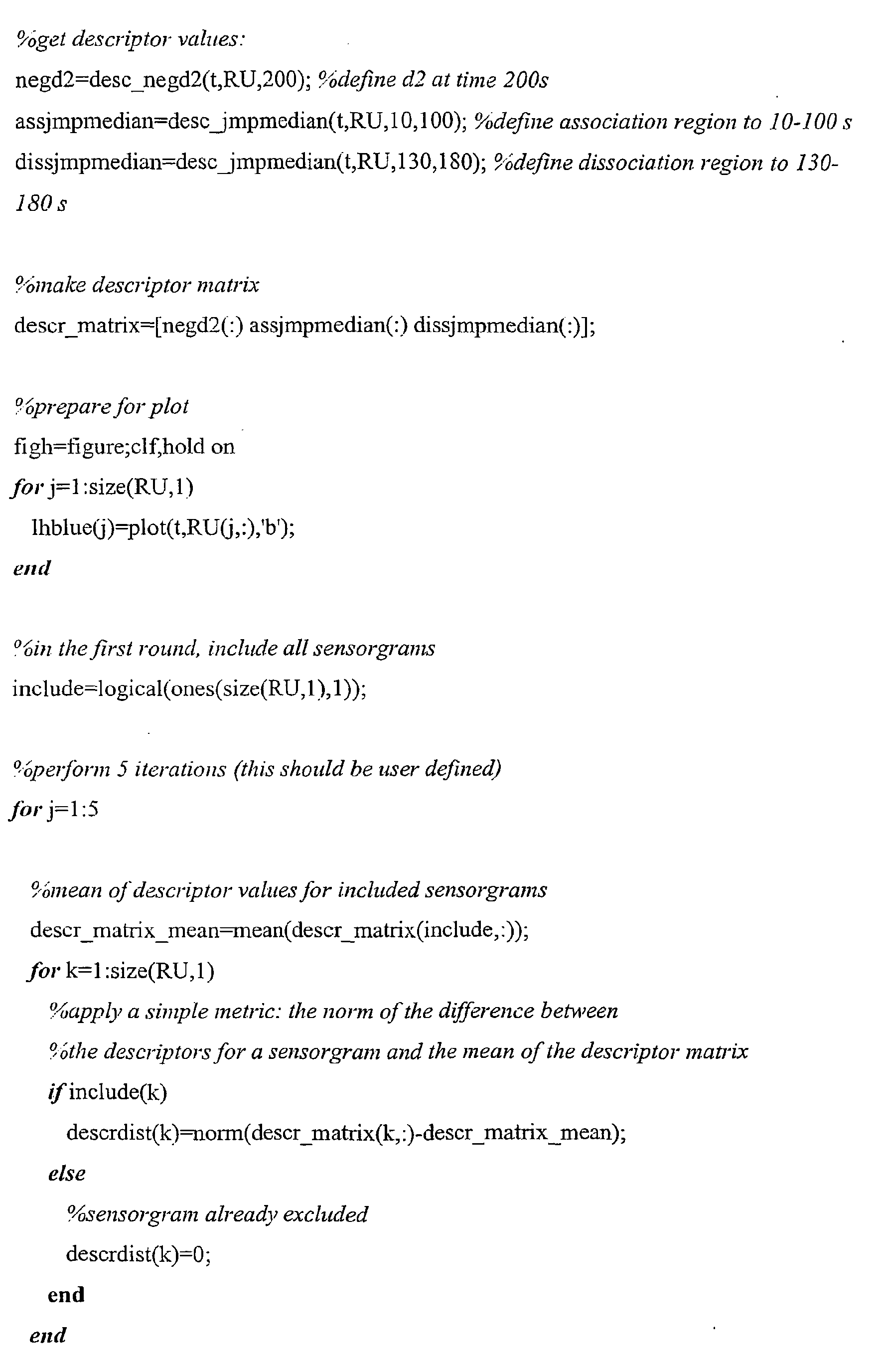

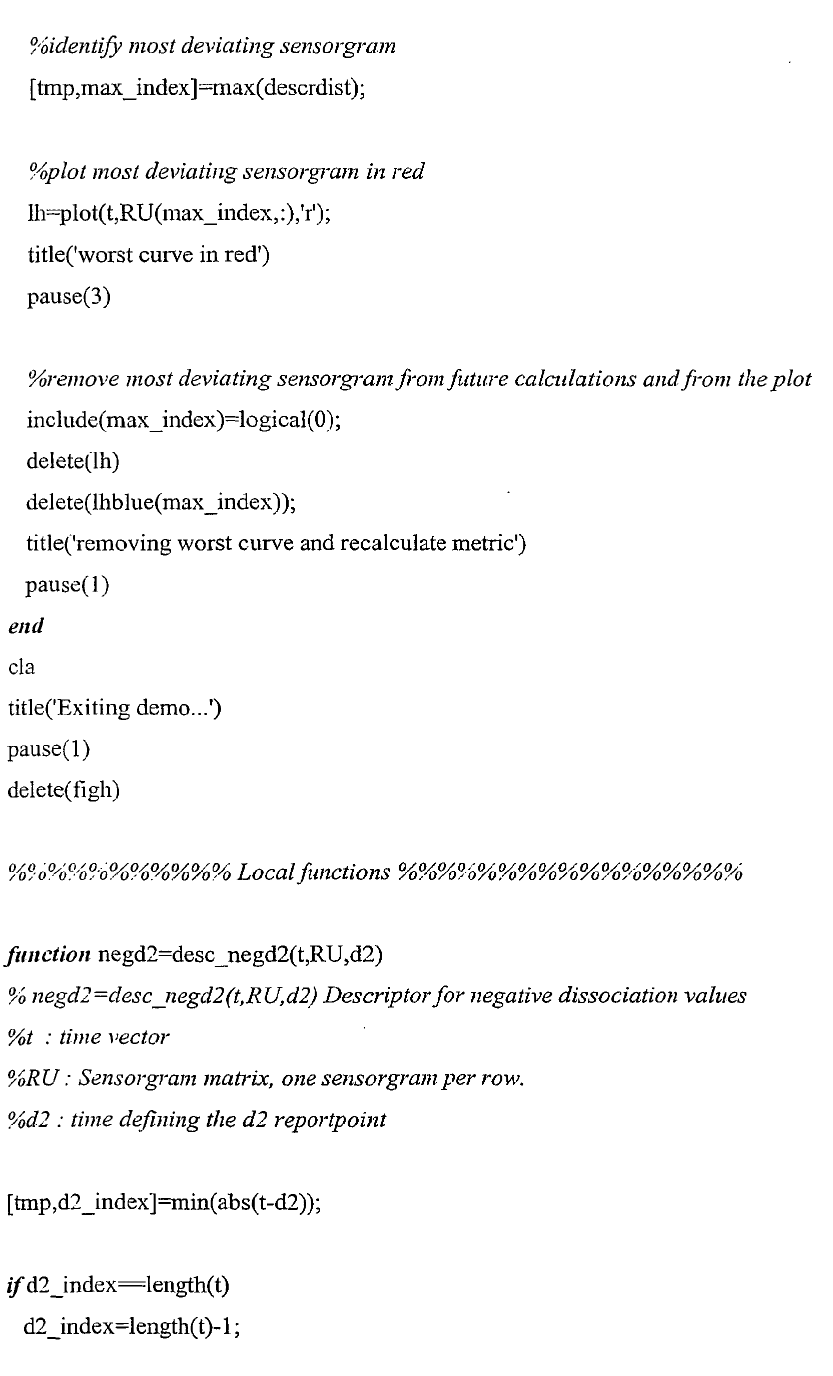

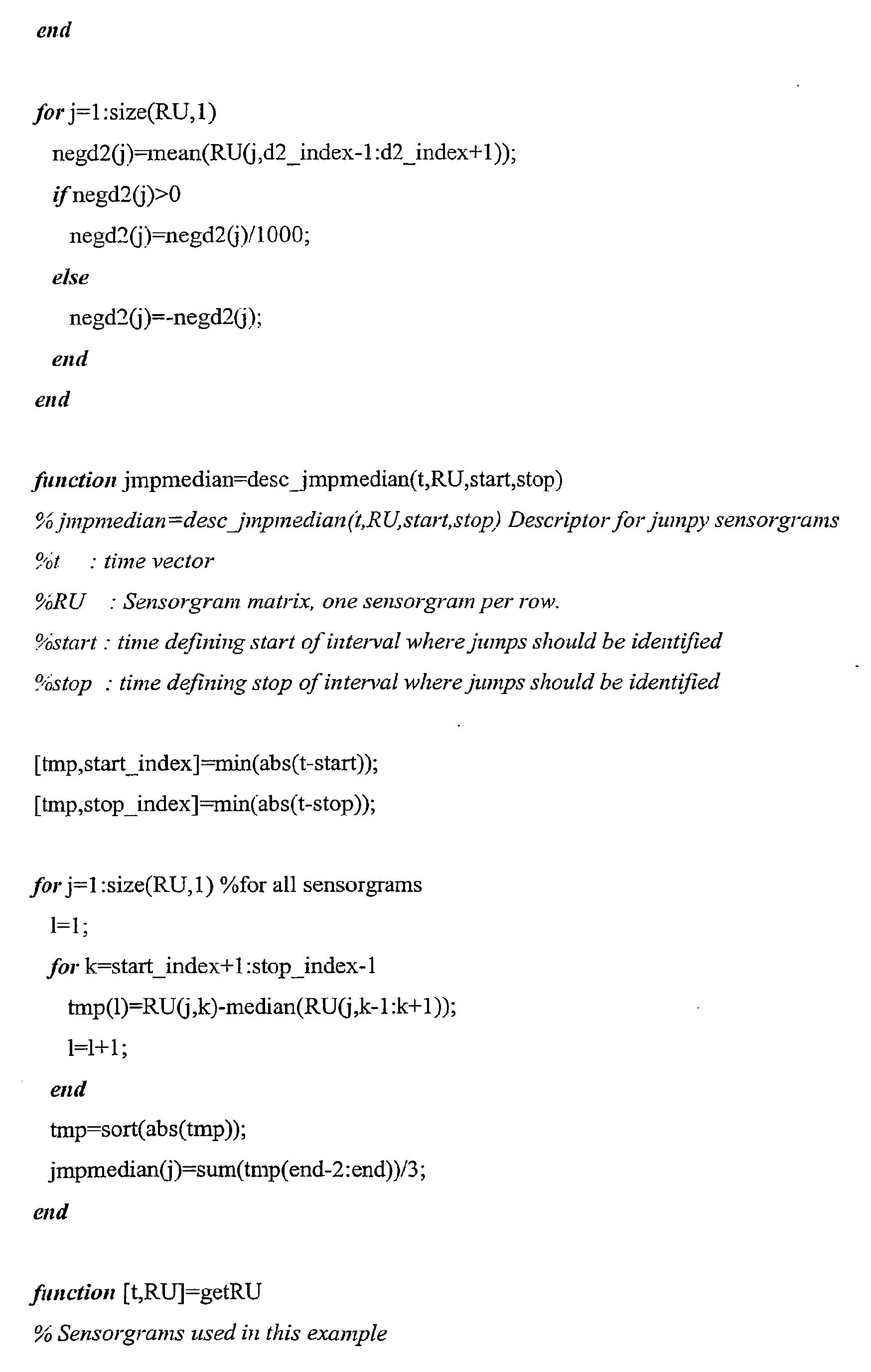

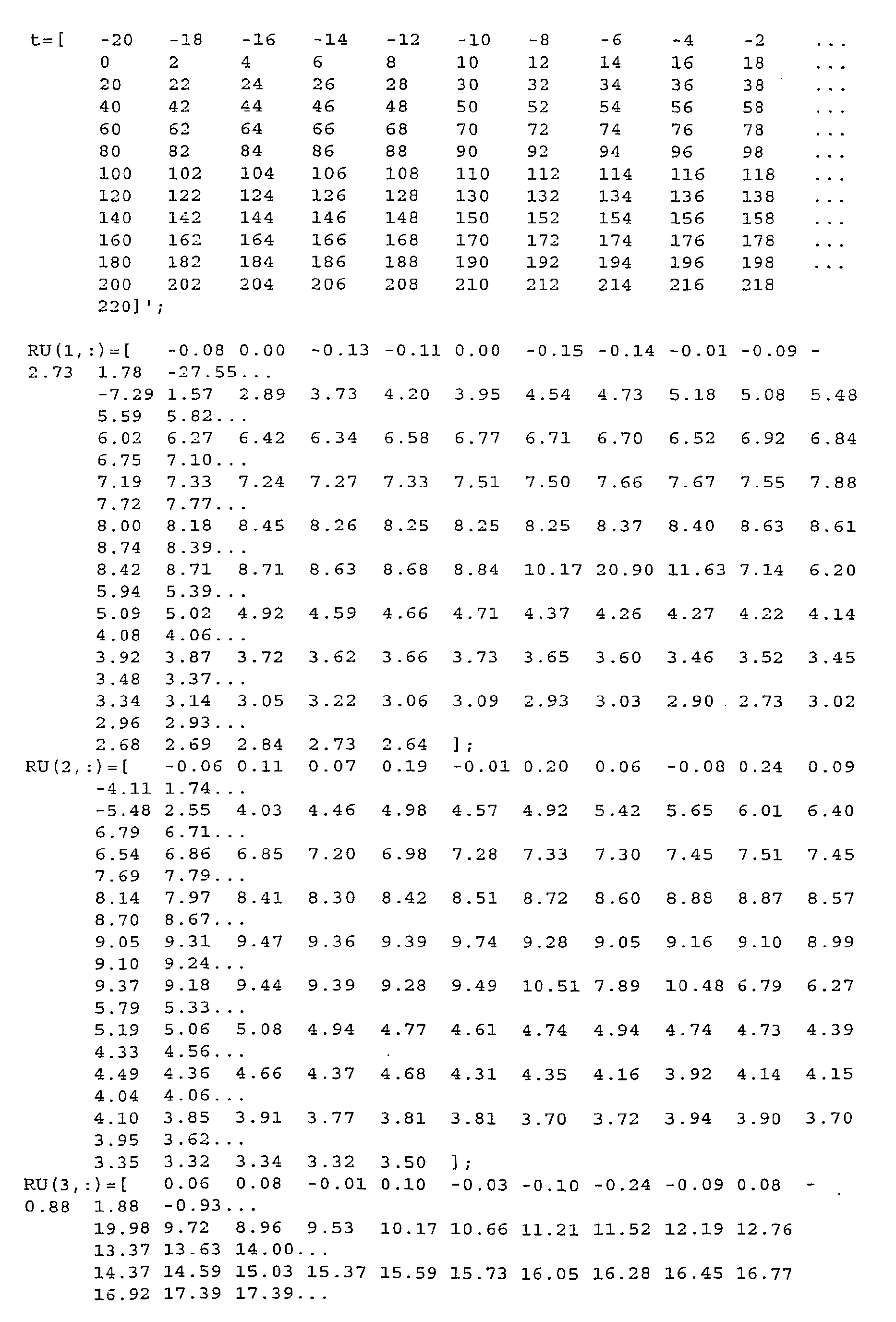

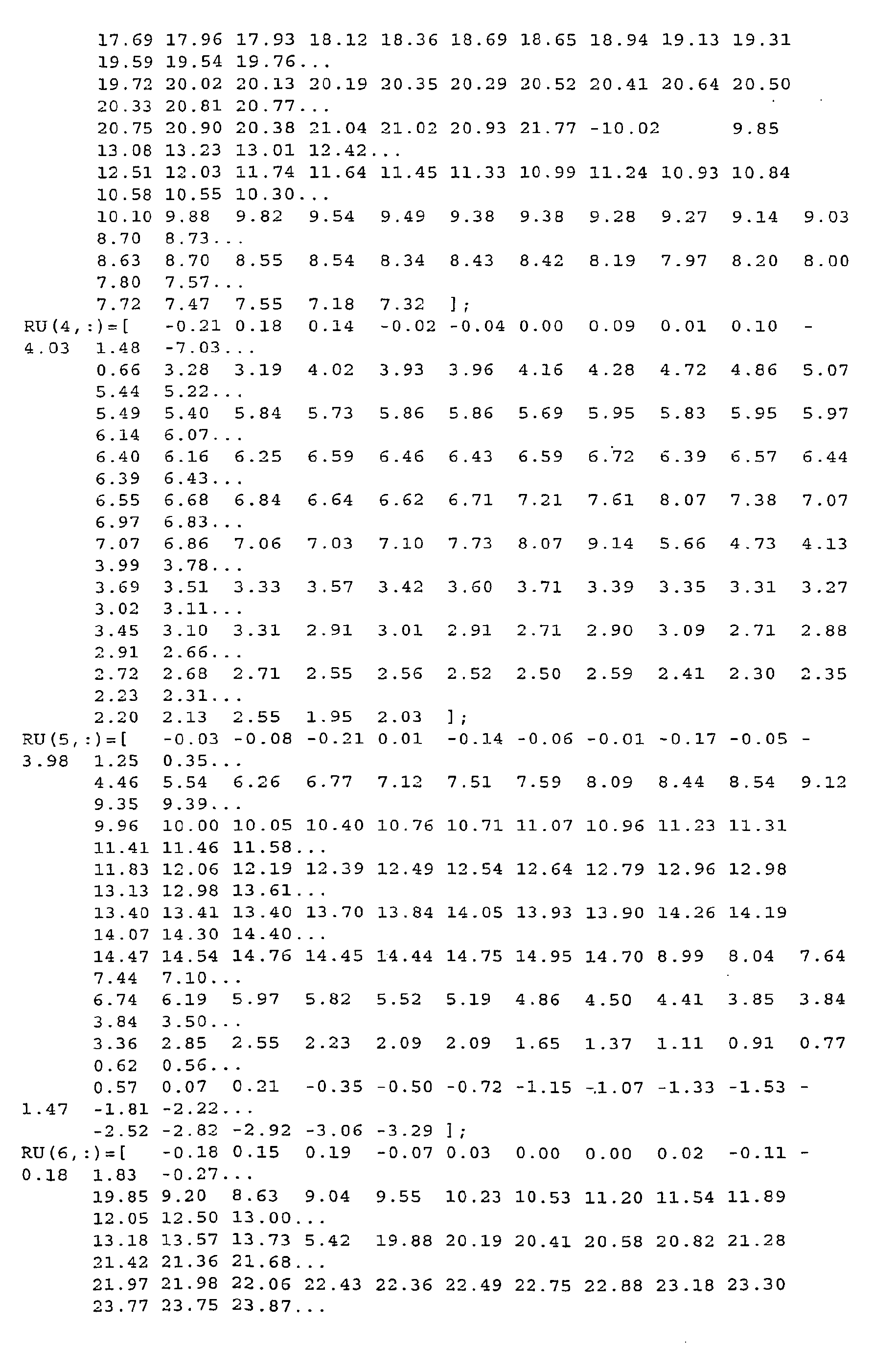

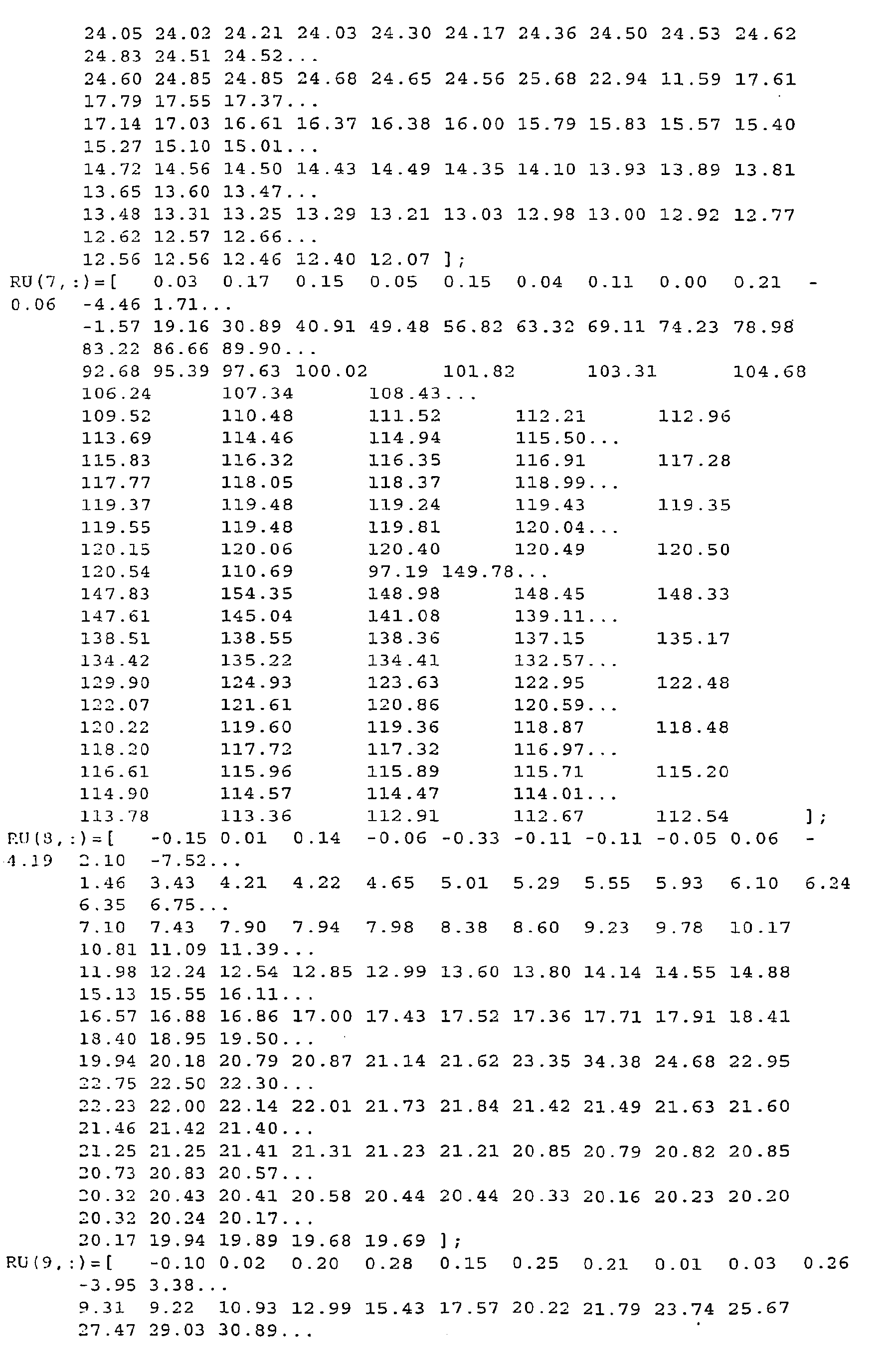

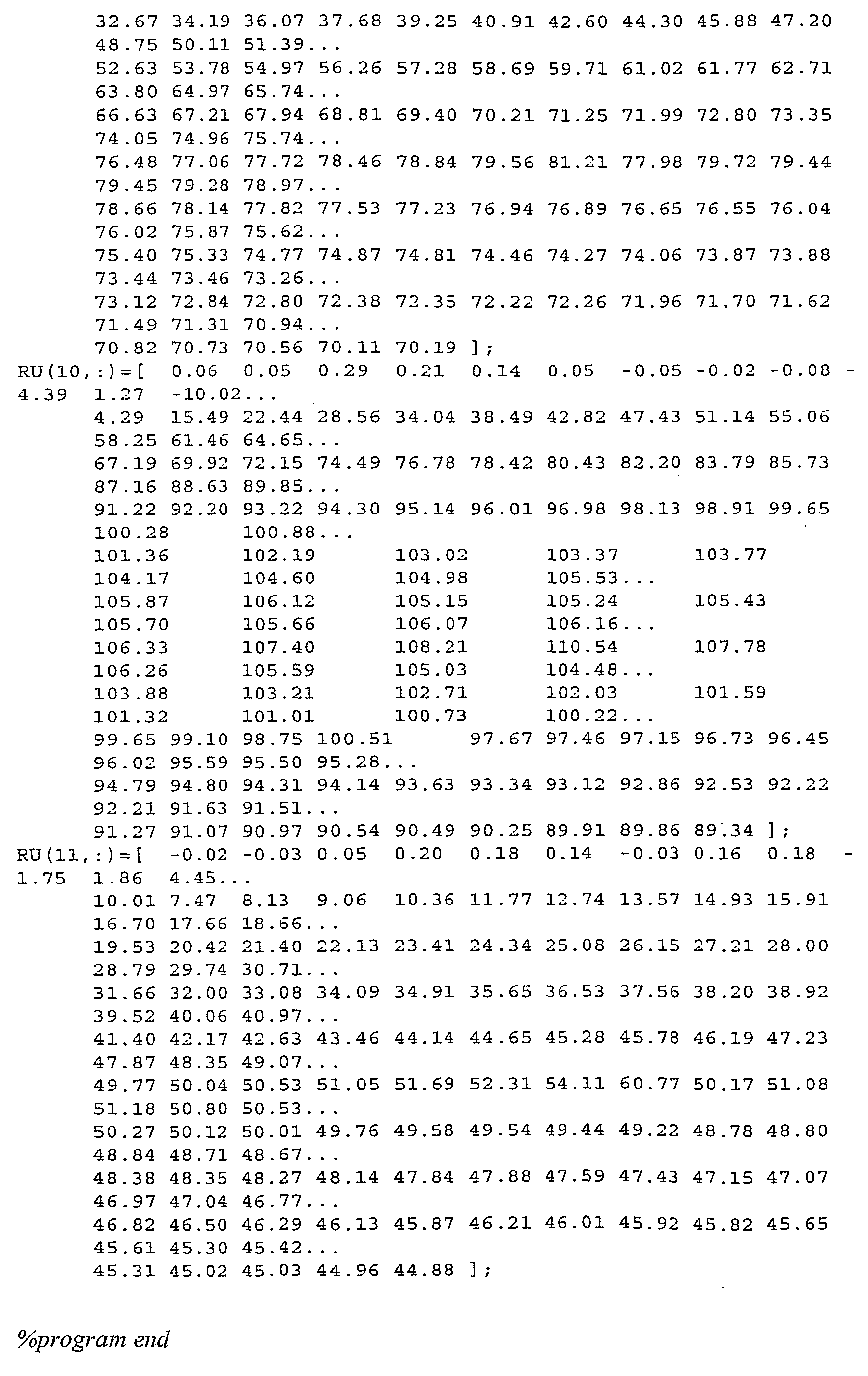

It is to be understood that the invention is not limited to the particular embodiments of the invention described above, but the scope of the invention will be established by the appended claims.

## Claims

1. A method of analysis, wherein molecular interactions at one or more sensing surface areas are detected and respective response curves representing the progress of each interaction with time are produced, and wherein a resulting set of response curves is subjected to a quality assessment procedure comprising the steps of:
a) selecting at least one quality-related parameter for the response curves, and for each different parameter defining at least one quality descriptor,
b) computing for each response curve in the set thereof, values for the different quality descriptors,
c) based on the values for the different quality descriptors, computing for each response curve a quality classification indicative of the quality of the response curve in relation to all response curves of the set,
d) selecting response curves having deviating quality classifications, and
e) subjecting the selected response curves to a validation procedure to determine whether a response curve or curves are to be rejected or not.

2. The method of claim 1, wherein steps c) to e) are repeated until no more response curves are rejected.

3. The method of claim 1 or 2, wherein step b) of claim 1 further comprises transforming the quality descriptor values for each response curve to a quality descriptor vector.

4. The method of claim 3, wherein a quality descriptor matrix is created from the quality descriptor vectors.

5. The method of any one of claims 1 to 4, wherein the descriptor values are normalized.

6. The method of any one of claims 1 to 5, wherein computing a quality classification in step c) of claim 1 comprises determining for each quality descriptor vector the difference between the vector and the rest of the quality descriptor vectors in the set of response curves.

7. The method of claim 6, wherein the determination of the differences between the vectors comprises determining a statistical measure of the distance from each quality descriptor vector to the rest of the quality descriptor vectors as a group.

8. The method of claim 6 or 7, wherein a difference vector is created from the computed differences.

9. The method of claim 6, 7 or 8, wherein step d) of claim 1 comprises sorting the quality vectors for the response curves in order of computed difference, and selecting a predetermined fraction of the response curves corresponding to those having the largest differences.

10. The method of any one of claims 1 to 5, wherein computing a quality classification in step c) of claim 1 comprises classifying the sensorgrams in groups of similar quality.

11. The method of any one of claims 1 to 5, wherein the quality classification is obtained by a neural network.

12. The method of any one of claims 1 to 5, wherein the quality classification is obtained by an expert system.

13. The method of any one of claims 1 to 12, wherein the validation procedure in step e) of claim 1 comprises subjecting selected response curves to an ocular inspection.

14. The method of any one of claims 1 to 13, wherein the validation procedure in step e) of claim 1 comprises determining which quality descriptor or descriptors that caused the quality classification of a sensorgram.

15. The method of any one of claims 1 to 14, wherein the validation procedure in step e) of claim 1 comprises identifying a time period or periods in the production of the sensorgrams to which a cluster of selected sensorgrams may be related.

16. The method of any one of claims 1 to 15, wherein the quality-related parameter or parameters on which the quality descriptors are based comprise at least one of an odd curve shape, carry-over between measurements, binding to a reference surface, and dissociation to value below zero.

17. The method of claim 16, wherein the odd curve shape is selected from sloping baseline, spikes, oscillations and jumps.

18. The method of any one of claims 2 to 17, wherein steps c) to e) of claim 1 are repeated with at least one different descriptor.

19. The method of any one of claims 2 to 18, wherein steps c) to e) of claim 1 are repeated with a different quality classification method.

20. The method of any one of claims 1 to 19, wherein a biosensor is used.

21. The method of claim 20, wherein the biosensor is based on evanescent wave sensing, particularly surface plasmon resonance.

22. An analytical system for detecting molecular binding interactions, comprising:
(i) a sensor device comprising at least one sensing surface, detection means for detecting molecular interactions at the at least one sensing surface, and means for producing response curves representing the progress of each interaction with time, and
(ii) data processing means for classifying the response curves with regard to quality, and wherein the data processing means perform steps a) to e) in claim 1 as defined in any one of claims 1 to 21..

23. A computer program comprising program code means for performing the quality assessment procedure of any one of claims 1 to 21 when the program is run on a computer.

24. A computer program product comprising program code means stored on a computer readable medium for performing the quality assessment procedure of any one of claims 1 to 21 when the program is run on a computer.

## Patentansprüche

1. Analyseverfahren, bei dem molekulare Wechselwirkungen an einem oder mehreren Sensorflächenbereichen nachgewiesen werden und jeweilige Reaktionskurven erstellt werden, die den Verlauf jeder Wechselwirkung über der Zeit darstellen, und bei dem eine sich daraus ergebende Gruppe von Reaktionskurven einem Qualitätsbeurteilungsverfahren unterzogen wird, das die Schritte umfasst:
a) Auswählen zumindest eines qualitätsbezogenen Parameters für die Reaktionskurven, und Festlegen zumindest eines Qualitätsdeskriptors für jeden unterschiedlichen Parameter,
b) Berechnen, für jede Reaktionskurve in der Gruppe derselben, von Werten für die unterschiedlichen Qualitätsdeskriptoren,
c) auf Grundlage der Werte für die unterschiedlichen Qualitätsdeskriptoren, Berechnen, für jede Reaktionskurve, einer Qualitätsklassifikation, die ein Indikator für die Qualität der Reaktionskurve im Verhältnis zu allen Reaktionskurven der Gruppe ist,
d) Auswählen von Reaktionskurven, die abweichende Qualitätsklassifikationen aufweisen, und
e) Unterziehen der ausgewählten Reaktionskurven einem Validierungsverfahren, um festzustellen, ob eine Reaktionskurve bzw. Reaktionskurven zu verwerfen ist bzw. sind oder nicht.

2. Verfahren nach Anspruch 1, bei dem die Schritte c) bis e) wiederholt werden, bis keine weiteren Reaktionskurven verworfen werden.

3. Verfahren nach Anspruch 1 oder 2, bei dem Schritt b) aus Anspruch 1 weiterhin das Umwandeln der Qualitätsdeskriptorwerte für jede Reaktionskurve zu einem Qualitätsdeskriptorvektor umfasst.

4. Verfahren nach Anspruch 3, bei dem eine Qualitätsdeskriptormatrix aus den Qualitätsdeskriptorvektoren erzeugt wird.

5. Verfahren nach einem der Ansprüche 1 bis 4, bei dem die Deskriptorwerte normalisiert werden.

6. Verfahren nach einem der Ansprüche 1 bis 5, bei dem das Berechnen einer Qualitätsklassifikation in Schritt c) aus Anspruch 1 umfasst, dass für jeden Qualitätsdeskriptorvektor die Differenz zwischen dem Vektor und dem Rest der Qualitätsdeskriptorvektoren in der Reaktionskurvengruppe festgestellt wird.

7. Verfahren nach Anspruch 6, bei dem die Feststellung der Differenzen zwischen den Vektoren umfasst, dass ein statistisches Maß der Distanz jedes Qualitätsdeskriptorvektors zum Rest der Qualitätsdeskriptorvektoren als Gruppe festgestellt wird.

8. Verfahren nach Anspruch 6 oder 7, bei dem ein Differenzvektor aus den berechneten Differenzen erzeugt wird.

9. Verfahren nach Anspruch 6, 7 oder 8, bei dem Schritt d) aus Anspruch 1 umfasst, dass die Qualitätsvektoren für die Reaktionskurven in Reihenfolge der berechneten Differenz sortiert werden, und dass ein vorgegebener Teil der Reaktionskurven entsprechend jenen mit den größten Differenzen ausgewählt wird.

10. Verfahren nach einem der Ansprüche 1 bis 5, bei dem das Berechnen einer Qualitätsklassifikation in Schritt c) aus Anspruch 1 das Klassifizieren der Sensorgramme in Gruppen ähnlicher Qualität umfasst.

11. Verfahren nach einem der Ansprüche 1 bis 5, bei dem die Qualitätsklassifikation durch ein neuronales Netzwerk erhalten wird.

12. Verfahren nach einem der Ansprüche 1 bis 5, bei dem die Qualitätsklassifikation durch ein Expertensystem erhalten wird.

13. Verfahren nach einem der Ansprüche 1 bis 12, bei dem das Validierungsverfahren in Schritt e) aus Anspruch 1 umfasst, dass ausgewählte Reaktionskurven einer visuellen Überprüfung unterzogen werden.

14. Verfahren nach einem der Ansprüche 1 bis 13, bei dem das Validierungsverfahren in Schritt e) aus Anspruch 1 umfasst, dass festgestellt wird, welcher Qualitätsdeskriptor bzw. welche Qualitätsdeskriptoren die Qualitätsklassifikation eines Sensorgramms bedingt hat bzw. haben.

15. Verfahren nach einem der Ansprüche 1 bis 14, bei dem das Validierungsverfahren in Schritt e) aus Anspruch 1 das Identifizieren eines Zeitraums bzw. von Zeiträumen in der Erstellung der Sensorgramme umfasst, zu dem bzw. denen ein Cluster ausgewählter Sensorgramme in Bezug stehen kann.

16. Verfahren nach einem der Ansprüche 1 bis 15, bei dem der bzw. die qualitätsbezogene(n) Parameter, auf dem bzw. denen die Qualitätsdeskriptoren beruhen, zumindest entweder eine merkwürdige Kurvenform oder Verschleppung zwischen Messungen oder Bindung an eine Referenzfläche oder Dissoziation auf einen Wert unter null umfasst bzw. umfassen.

17. Verfahren nach Anspruch 16, bei dem die merkwürdige Kurvenform gewählt ist aus schräger Basislinie, Spitzen bzw. Spikes, Schwankungen und Sprüngen.

18. Verfahren nach einem der Ansprüche 2 bis 17, bei dem die Schritte c) bis e) aus Anspruch 1 mit zumindest einem anderen Deskriptor wiederholt werden.

19. Verfahren nach einem der Ansprüche 2 bis 18, bei dem die Schritte c) bis e) aus Anspruch 1 mit einem anderen Qualitätsklassifikationsverfahren wiederholt werden.

20. Verfahren nach einem der Ansprüche 1 bis 19, bei dem ein Biosensor eingesetzt wird.

21. Verfahren nach Anspruch 20, bei dem der Biosensor auf Evaneszentwellen-Sensing, insbesondere Oberflächenplasmonenresonanz, beruht.

22. Analytisches System zum Nachweis molekularer Bindungswechselwirkungen, umfassend:
(i) eine Sensorvorrichtung, umfassend zumindest eine Sensorfläche, Nachweismittel zum Nachweis molekularer Wechselwirkungen an der zumindest einen Sensorfläche und Mittel zur Erstellung von Reaktionskurven, die den Verlauf jeder Wechselwirkung über der Zeit darstellen, und
(ii) Datenverarbeitungsmittel zur Klassifizierung der Reaktionskurven in Bezug auf Qualität, und wobei die Datenverarbeitungsmittel die Schritte a) bis e) in Anspruch 1 durchführen, wie in einem der Ansprüche 1 bis 21 definiert.

23. Computerprogramm, das Programmcodemittel umfasst, um das Qualitätsbeurteilungsverfahren nach einem der Ansprüche 1 bis 21 vorzunehmen, wenn das Programm auf einem Computer ausgeführt wird.

24. Computerprogrammprodukt, das auf einem computerlesbaren Medium gespeicherte Programmcodemittel umfasst, um das Qualitätsbeurteilungsverfahren nach einem der Ansprüche 1 bis 21 vorzunehmen, wenn das Programm auf einem Computer ausgeführt wird.

## Revendications

1. Procédé d'analyse, dans lequel des interactions moléculaires au niveau d'une ou plusieurs zones superficielles de détection sont détectées et des courbes de réponse respectives représentant la progression de chaque interaction en fonction du temps sont produites, et dans lequel un jeu de courbes de réponse résultant est soumis à une procédure d'évaluation de qualité comprenant les étapes de :
a) sélection d'au moins un paramètre associé à la qualité pour les courbes de réponse, et pour chaque différent paramètre, définition d'au moins un descripteur de qualité,
b) calcul, pour chaque courbe de réponse dans le jeu de celles-ci, de valeurs pour les différents descripteurs de qualité,
c) sur la base des valeurs pour les différents descripteurs de qualité, calcul pour chaque courbe de réponse d'une classification de qualité représentative de la qualité de la courbe de réponse par rapport à l'ensemble des courbes de réponse du jeu,
d) sélection de courbes de réponse présentant des écarts de classification de qualité, et
e) d'application sur les courbes de réponse sélectionnées d'une procédure de validation afin de déterminer si une courbe ou des courbes de réponse doivent être rejetées ou non.

2. Procédé selon la revendication 1, dans lequel les étapes c) à e) sont répétées jusqu'à ce que plus aucune courbe de réponse n'est rejetée.

3. Procédé selon la revendication 1 ou 2, dans lequel l'étape b) de la revendication 1 comprend, en outre, la transformation des valeurs de descripteur de qualité de chaque courbe de réponse en un vecteur de descripteur de qualité.

4. Procédé selon la revendication 3, dans lequel une matrice de descripteur de qualité est créée à partir des vecteurs de descripteur de qualité.

5. Procédé selon l'une quelconque des revendications 1 à 4, dans lequel les valeurs de descripteur sont normalisées.

6. Procédé selon l'une quelconque des revendications 1 à 5, dans lequel le calcul d'une classification de qualité à l'étape c) de la revendication 1 comprend la détermination, pour chaque vecteur de descripteur de qualité, de la différence entre le vecteur et le reste des vecteurs de descripteur de qualité dans le jeu de courbes de réponse.

7. Procédé selon la revendication 6, dans lequel la détermination des différences entre les vecteurs comprend la détermination d'une mesure statistique de la distance entre chaque vecteur de descripteur de qualité et le reste des vecteurs de descripteur de qualité en tant que groupe.

8. Procédé selon la revendication 6 ou 7, dans lequel un vecteur de différence est créé à partir des différences calculées.

9. Procédé selon la revendication 6, 7 ou 8, dans lequel l'étape d) de la revendication 1 comprend le tri des vecteurs de qualité pour les courbes de réponse suivant l'ordre de différence calculée, et la sélection d'une fraction prédéterminée des courbes de réponse respectives correspondant à celles présentant les plus grandes différences.

10. Procédé selon l'une quelconque des revendications 1 à 5, dans lequel le calcul d'une classification de qualité à l'étape c) de la revendication 1 comprend la classification des graphes de détection en groupes de qualité similaire.

11. Procédé selon l'une quelconque des revendications 1 à 5, dans lequel la classification de qualité est obtenue par un réseau de neurones.

12. Procédé selon l'une quelconque des revendications 1 à 5, dans lequel la classification de qualité est obtenue par un système expert.

13. Procédé selon l'une quelconque des revendications 1 à 12, dans lequel la procédure de validation de l'étape e) de la revendication 1 comprend l'exposition des courbes de réponse sélectionnées à une inspection oculaire.

14. Procédé selon l'une quelconque des revendications 1 à 13, dans lequel la procédure de validation de l'étape e) de la revendication 1 comprend la détermination du descripteur ou des descripteurs de qualité qui provoquent la classification de qualité d'un graphe de détection.

15. Procédé selon l'une quelconque des revendications 1 à 14, dans lequel la procédure de validation de l'étape e) de la revendication 1 comprend l'identification d'une période ou de périodes de temps au cours de la production des graphes de détection auxquelles un groupe de graphes de détection sélectionné peut être associé.

16. Procédé selon l'une quelconque des revendications 1 à 15, dans lequel le paramètre ou les paramètres associés à la qualité sur lesquels sont basés les descripteurs de qualité comprennent au moins l'un d'une forme courbe impaire, d'un report entre des mesures, d'une liaison à une surface de référence, et d'une dissociation par rapport à une valeur inférieure à zéro.

17. Procédé selon la revendication 16, dans lequel la forme de courbe impaire est sélectionnée à partir d'une ligne de base inclinée, de pics, d'oscillations et d'échelons.

18. Procédé selon l'une quelconque des revendications 2 à 17, dans lequel les étapes c) à e) de la revendication 1 sont répétées avec au moins un descripteur différent.

19. Procédé selon l'une quelconque des revendications 2 à 18, dans lequel les étapes c) à e) de la revendication 1 sont répétées avec un procédé de classification de qualité différent.

20. Procédé selon l'une quelconque des revendications 1 à 19, dans lequel un biocapteur est utilisé.

21. Procédé selon la revendication 20, dans lequel le biocapteur est basé sur la détection d'onde évanescente, en particulier, la résonance de plasmon de surface.

22. Dispositif analytique de détection d'interactions de liaison moléculaire, comprenant :
(i) un dispositif de détection comprenant au moins une surface de détection, des moyens de détection destinés à détecter des interactions moléculaires au niveau de la au moins une surface de détection, et des moyens destinés à produire des courbes de réponse représentant la progression de chaque interaction en fonction du temps, et
(ii) un moyen de traitement de données destiné à assurer la classification des courbes de réponse par rapport à la qualité, et
dans lequel le moyen de traitement de données exécute les étapes a) à e) de la revendication 1 selon l'une quelconque des revendications 1 à 21.

23. Programme informatique comprenant un moyen formant code informatique destiné à exécuter la procédure d'évaluation de qualité selon l'une quelconque des revendications 1 à 21, lorsque le programme est exécuté sur un ordinateur.

24. Produit formant programme informatique comprenant un moyen formant code informatique mémorisé sur un support pouvant être lu par ordinateur afin d'exécuter la procédure d'évaluation de qualité selon l'une quelconque des revendications 1 à 21 lorsque le programme est exécuté sur un ordinateur.
